Europäisches Patentamt

European Patent Office

Office européen des brevets

(19)

(11) Publication number: **0 348 517**
A1

# EUROPEAN PATENT APPLICATION

published in accordance with Art. 158(3) EPC

(21) Application number: 88910122.6

(22) Date of filing: 29.11.88

Data of the international appli-
cation taken as a basis:

(86) International application number:
PCT/JP 88/01205

(87) International publication number:
WO 89/05153 (15.06.89 89/13)

(51) Int. Cl.⁴: **A 61 K 37/32**

(30) Priority: 03.12.87 JP 304422/87
05.10.88 JP 249970/88

(43) Date of publication of application: 03.01.90
Bulletin 90/1

(84) Designated Contracting States: CH DE FR GB LI

(71) Applicant: THE CALPIS FOOD INDUSTRY CO., LTD.,
20-3, Ebisu-Nishi 2-chome, Shibuya-ku Tokyo 150 (JP)

(72) Inventor: OHMURA, Kazutaka, 3-3-14, Kitakashiwa
Kashiwa-shi, Chiba-ken (JP)
Inventor: NAKAMURA, Yasunori, 1856,
Shinyoshida-chou Kouhoku-ku, Yokohama-shi
Kanagawa-ken (JP)
Inventor: NOMURA, Kaoru, 12-15, 1-chome
Kichijoujihigashi-chou, Musasino-shi Tokyo (JP)
Inventor: UJIHARA, Makoto, Puraza-Kojimaya 303 32-1,
Wakamatsu-chou, Shinjuku-ku Tokyo (JP)
Inventor: TOYOSHIMA, Satoshi,
Tendai-jutaka 3-102 Tendai 1-9, Chiba-shi Chiba-ken
(JP)
Inventor: YAMAMOTO, Kazuo, 4-28-9-104, Hongou,
Bunkyo-ku Tokyo (JP)
Inventor: OSAWA, Toshiaki, 1-9-13, Akatsukashin
Itabashi-ku, Tokyo (JP)
Inventor: SHIZUME, Kazuo, 3-28, Yoyogi Shibuya-ku,
Tokyo (JP)

(74) Representative: Thomsen, Dieter, Dr.rer.nat.,
Kaiser-Ludwig-Platz 6, D-8000 München 2 (DE)

(54) KIDNEY GROWTH ACCELERATOR.

(57) A kidney growth accelerator based on a finding that a lu-
teinizing hormone or its isoform has a kidney growth accelerat-
ing activity. Administration of the luteinizing hormone or its
isomer is expected to activate a kidney suffering reduction in
the number of kidney cells or suffering deterioration of kidney
functions.

EP 0 348 517 A1

0 348 517

## RENAL GROWTH PROMOTER

This invention relates to a renal growth promoter and a process for the production of the same.

More particularly, it relates to a renal growth promoter which comprises luteinizing hormone having an activity of promoting renal growth or an isomer thereof as an active ingredient.

The present invention is based on the finding that luteinizing hormone or an isomer thereof has an effect of promoting renal growth. Thus it is expected that the administration of the renal growth promoter of the present invention would increase the number of renal cells or enhance a renal function to thereby activate the kidney suffering from a decrease in renal cells or a lowering in the renal function, which considerably contributes to medical and pharmacological fields.

Background Art

It has never been known that luteinizing hormone or an isomer thereof has an effect of promoting renal growth.

The present inventors concentrated their attention to the fact that hypophysectomized mice would show a significant decrease in the renal weight and considered that there might be some factor controlling renal growth in the pituitary. Thus they attempted to find out a renal growth factor present in pituitary. Consequently they obtained a fraction containing a hormone-like component promoting renal growth from the pituitary and named the unisolated component promoting renal growth RGF (or renotropin) (cf. Nippon Rinsho, $\underline{44}$ (1), separate issue, 84 - 88 (Jan. 1986)).

Brief Description of the Drawing

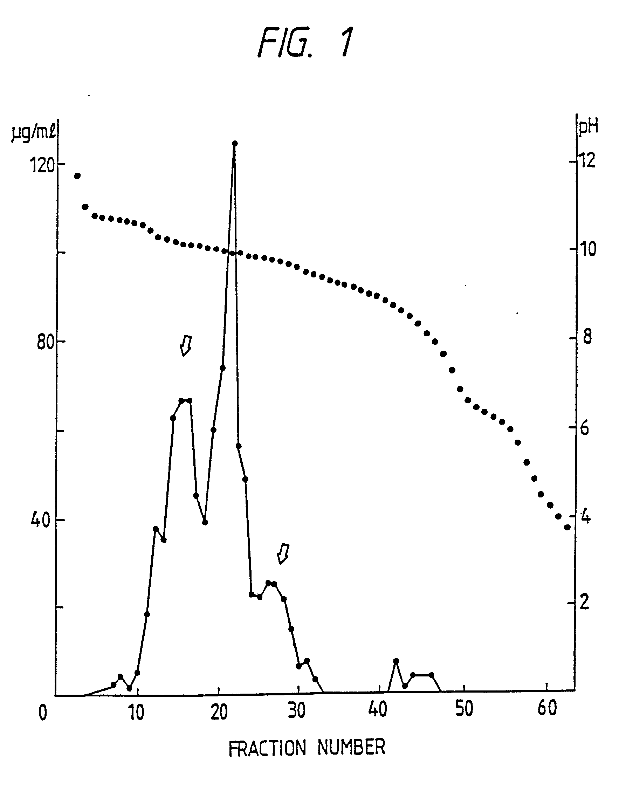

Fig. 1 shows the protein content of each fraction obtained by isoelectric focusing of a crude renal growth factor in Example 1.

Problems to be solved by the Invention

However it has been found that the RGF-containing fraction thus obtained is a crude product which shows an unstable activity and contains various contaminants having antagonistic effects.

In order to obtain the RGF as a single component, the present inventors have further conducted studies. As a result, they have unexpectedly found that a single band of an active ingredient can be obtained by fractionating and purifying the crude RGF-containing fraction obtained above according to the difference in isoelectric point.

The analysis of this single band and the determination of the primary structure thereof have surprisingly revealed that it is an isomer of luteinizing hormone, and the fact that the RGF-containing fraction is a mixture of isomers of luteinizing hormone, was also found.

It is known that luteinizing hormone is not a single compound but glycoprotein comprising a plurality of isomers. This is caused by the microstructural polymorphism thereof.

It was reported that ovine luteinizing hormone had heterogeneous amino-terminal sequences on the α subunit and heterogeneous carboxyl-terminal sequences on the β subunit (D.N. Ward, Int. J. Peptide Protein Res., 27, 70 - 78 (1986)). It was further reported that human luteinizing hormone was divided into seven peaks by column electrofocusing (cf. M. Yano, Acta Obst Gynaec Jpn , 37 (5), 703 - 712 (1985)).

As a matter of course, it has never been known that luteinizing hormone and each isomer thereof would have an effect of promoting renal growth.

Subsequent studies by the present inventors have revealed that the highest activity of promoting renal growth is achieved by an isomer of luteinizing hormone wherein a subunit $\alpha_3$ is bound to another subunit $\beta_3$.

The present invention first relates to a renal growth promoter comprising luteinizing hormone or an isomer thereof as an active ingredient. It is preferable that said isomer of luteiniizng hormone comprises a subunit $\alpha_3$ bound to another subunit $\beta_3$.

The present invention further provides a process for production of the isomer of luteinizing hormone having an activity of promoting renal growth which comprises fractionating and purifying a crude renal growth factor of luteinizing hormone (mixture of isomers), according to the difference in isoelectric point.

In accordance with the present invention, the amount of the neutral glyco-chain component among the glyco-chain component in β subunit in each isomer of luteinizing hormone as herein isolated was measured, while the renal growth promoting activity thereof was also measured.

The present invention has revealed the fact that the isomer containing 30 % or more neutral glyco-chain component in the glyco-chain component composition of the β subunit of luteinizing hormone has an excellent renal growth promoting activity. Of course, it has not hitherto been known at all that the isomer possesses a renal growth promoting activity.

The present invention relates to a luteinizing hormone which contains 30 % or more neutral glyco-chain component in the glyco-chain component composition of the β subunit of luteinizing hormone, and it also relates to a renal growth promoter containing, as an active ingredient, the luteinizing hormone which contains 30 % or more neutral glyco-chain component in the glyco-chain component composition of the β-subunit of luteinizing hormone.

Further, the present invention has also revealed the fact that the isomer containing 18 % or more neutral glyco-chain component in the glyco-chain component composition of the β subunit of luteinizing hormone has an excellent renal growth promoting activity. Of course, it has not hitherto been known at all that the isomer possesses a renal growth promoting activity.

The present invention relates to a luteinizing hormone which contains 18 % or more neutral glyco-chain component in the glyco-chain component composition of the β subunit of luteinizing hormone, and it also relates to a renal growth promoter containing, as an active ingredient, the luteinizing hormone which contains 18 % or more neutral glyco-chain component in the glyco-chain component composition of the β subunit of luteinizing hormone.

The fractionation and purification treatment according to the difference in isoelectric point as described herein means a treatment whereby ampholytes such as proteins are fractionated from each other according to the inherent isoelectric point of each material and then separately collected. It may be carried out by, for example, chromatofocusing or electrofocusing.

As the crude renal growth factor to be used as the starting material in the present invention, crude luteinizing hormone extracts obtained from urine, blood or organs of mammals such as mouse, sheep or swine may be employed. Alternatively, a crude product obtained from a culture system which is artificially prepared through genetic engineering may be used therefor. Further a material obtained from a synthetic chemical reaction system may be employed therefor. Anyway, a mixture of an isomer of luteinizing hormone having the effect of promoting renal growth with those having no such an effect is employed as the starting material.

For example, the crude renal growth factor is obtained from an organ in the following manner. An aqueous solution of ammonium sulfate is added to the pituitary and the resulting mixture is homogenized and centrifuged. The supernatant is purified and lyophilized. Then the lyophilized material is dissolved and purified by a series of treatments, for example, SP Sephadex chromatography, Sephadex G100 chromatography, CM celluose chromatography, Con. A chromatography and Sephadex G100 chromatography to thereby give a crude renal growth factor of luteinizing hormone comprising a mixture of plural isomers.

The crude renal growth factor thus obtained can directly be used as a renal growth promoter, as having the renal growth promoting activity of 112 % as shown in Experimental Example to follow hereunder.

The crude renal growth factor obtained may be subjected to fractionation and purification treatment according to the difference in isoelectric point, whereby each isomer having the renal growth promoting activity can be obtained as a single band.

In general, luteinizing hormone is a glycoprotein, which is an associated substance composed of two protein subunits of α subunit and β subunit, and it has already been known that α subunit has glyco-chains as bonded to two asparagins and β subunit has a glyco-chain as bonded to one asparagin.

Luteinizing hormone is produced in a pituitary gland in a living body and is characteristically differentiated from other similar substances of glycoproteins, such as chorionic gonadotropic hormone or the like, which are bio-synthesized in other organs, in that the glyco-chain terminals in the former include sulfated ones, and sialated ones in some degree.

The glyco-chain of luteinizing hormone is known as a typical form of a so-called asparagine-bonded glyco-chain. The saccharide components to constitute the glyco-chain are essentially monosaccharides including mannose, fructose galactose, glucose, N-acetylglucosamine and N-acetylgalactosamine. All the monosaccharides constituting the said glyco-chain skeletons are neutral. When a sulfuric acid and/or a sialic acid is(are) added to any position of the said glyco-chain skeleton, the resulting glyco-

-8-

chain is acidified as a whole. The number of the acid is generally one or two in many cases, but it may be more than two as the case may be. In the present invention, when one or more of sulfuric acid and/or sialic acid is(are) added to the glyco-chain, the resulting glyco-chain is called an acidic glyco-chain, while that having neither the acid group nor the acid is called a neutral glyco-chain.

It is well known that the typical asparagine-bonded glyco-chain as a glyco-chain structure of glycoproteins includes various structural modifications. Making a general classification, mannose-rich type, complex type and hybrid type structures are well known. On the basis of these basical types, further more modifications have been reported. There may be numerous modifications, for example, to be caused by presence or absence of fructose or bisect N-acetylglucosamine, presence or absence of N-acetylgalactosamine in place of galactose, or presence of single chain, double chain, triple chain or quadruple chain from mannose as the branching point, as well as presence or absence of sialic acid or sulfuric acid added. Typical examples of the heterogenity can be found in many literatures. Unexceptionally there are many of the said heterogeneous modification also in the glyco-chains added to luteinizing hormone.

In accordance with the present invention, the luteinizing hormone containing 30 % or more neutral glyco-chain component in the β subunit thereof has first newly been fractionated and this has been found to be a luteinizing hormone having an excellent renal cell DNA synthesis promoting activity. It is one new finding.

In accordance with the present invention, the luteinizing hormone containing 18 % or more neutral glyco-chain component in the β subunit thereof has next also newly been fractionated and this has also been found to be a luteinizing hormone having an excellent renal cell DNA synthesis promoting activity. It is another new finding.

Accordingly, each substance with a single band obtained by fractionation and purification of the crude renal growth factor according to the difference in isoelectric point is analyzed to measure the glyco-chain component content in each substance, and the fraction having the neutral glyco-chain component in an amount of 30 % or more or having the same in an amount of 18 % or more is fractionated, whereby luteinizing hormone (isomer) having a higher renal growth promoting activity can be obtained.

The material having an activity of promoting renal growth obtained as a single band may be separated into constituting subunits $\alpha$ and $\beta$. As a matter of course, the renal growth promoting activity would disappear after dividing the material into these subunits.

The separation may be carried out by, for example, reverse phase liquid chromatography. In the separation pattern, the subunits $\alpha$ and $\beta$ show each a plurality of peaks depending on the length of amino acids' chain. It has been confirmed that these peaks correspond to the several shorter amino acids' chains obtained by cutting the subunits $\alpha$ and $\beta$, which chains had been reported previously.

Although a material wherein a subunit $\alpha_3$ is bound to another subunit $\beta_3$ has a slight luteinizing hormone activity, it is different from other isomers of luteinizing hormone in that it has a remarkable renal growth promoting activity.

The subunits $\alpha_3$ and $\beta_3$ are those which suffer from the least cuttings. Among various combinations of the subunits which are bound to each other again, the one having the subunits $\alpha_3$ and $\beta_3$ showed the highest activity of promoting renal growth.

Measurement of glyco-chain component content can be carried out by, for example, the following method.

Method of Measurement of Glyco-chain Component Content:

For cutting the bond between the asparagine residues in glyco-chain, any of chemical method and enzymatical method can be employed. However, the former is better, since this is free from cutting failure. In the present invention, hydrazine decomposition method which is known as illustrated in Matsushima et al, Bull. Chem. Soc. Jpn., 30, 48, '57 and in Z. Yosizawa et al, Biochim. Biophys. Acta, 121, 417, '66, was employed. As is known from S. Takasaki et al, Method in Enzynology (edited by V. Ginsburg, Academic Press, New York, Vol. 83, 263, '82), the reducing terminal of the glyco-chain as cut out from asparagine residue by conventional method is labeled with tritium and then used in the subsequent quantitative analysis.

The tritium-labeled glyco-chain is separated and fractionated by anion-exchange chromatography such as DEAE-Sephacel® into a neutral glyco-chain fraction and an acid glyco-chain fraction, each of which is quantitatively determined. From the value thus determined, the neutral glyco-chain component content as referred to herein can be obtained as percentage.

However, a measurement error of about 10 % or so of the value measured may be experimentally considered, which would be derived from the depreciation of the reactants during chemical reaction operation as well as from the depreciation of the products during column chromatography operation.

Experimental Example

A pertinent amount of the lyophilized powder of each fraction of pI 10.32 to 10.15 and pI 10.15 to 9.89 obtained in Example 1 was used as a sample, which was subjected to high performance liquid chromatography to fractionate into subunits α and β.

Using reverse phase column, each sample was fractionated with a linear gradient system of water-organic solvent (acetonitrile/isopropanol = 3/7) in the presence of 0.1 % trifluoroacetic acid, whereby it was fractionated into α subunit as relatively early eluted out and β subunit as eluted out late. Next, each of the fractions was dried and powdered under reduced pressure.

About 1 mg of the β subunit powder of each fraction of pI 10.32 to 10.15 and pI 10.15 to 9.89 was weighed in a test tube and 1 ml of anhydrous hydrazine was added thereto. This was heated at 105°C for 4 hours in the presence of hydrazine sulfate as a catalyst. After the reaction, hydrazine was evaporated out under a reduced pressure. From 0.5 to 1 ml of an aqueous 4.5 M sodium acetate solution was added to the resulting residue, and acetic anhydride was added thereto several times, totaling 100 µl, with stirring. After being allowed to stand at room temperature for 30 minutes, it was desalted by treatment in Sephadex G25® column to obtain an oligosaccharide fraction. Next, this was concentrated under a reduced pressure to dryness.

Subsequently, 100 µl of water was added to the dry solid and dissolved and an excess amount (2.5 mCi) of NaB$^3$H$_4$ (in the form of 0.1 M sodium hydroxide solution) was added thereto to reduce the solid for 16 hours at room temperature. After the reaction, 0.5 ml of 1 M acetic acid was added to the reaction mixture, which was then desalted by treatment in Dowex 50W-X8 column. The eluted fraction was concentrated to dryness. Next, in order to remove boric acid from the solid, several milliliters of methanol was added thereto and subjected to reduced pressure three times. Then this was subjected to paper chromatography using a developer solvent of ethyl acetate/acetic acid/formic acid/water (18/3/1/4, v/v) and Whatman 3MM filter paper, whereupon the radioactive site was extracted with water to recover the glyco-chain.

Next, the sample was dissolved in 2 mM Tris-HCl buffer (pH 7.4) and then subjected to DEAE-Sephacel® (Pharmacia) column as equilibrated with the same buffer, whereupon the passed-through fraction was collected. This was neutral glyco-chain fraction (N). The sodium

-14-

chloride concentration in the same buffer was elevated up to 100 mM by linear gradient, and the fraction as eluted at about 25 mM, 50 mM or 75 mM or so was called acid glyco-chain fraction (A-1), (A-2) or (A-3), respectively.

The strength of the radioactivity of each fraction was measured by conventional method where the respective rots were varied.

The value of each of N, A-1, A-2 and A-3 of each sample was shown in Tables 1 and 2 below, as percentage.

Table 1

|  | N | A-1 | A-2 | A-3 | Total |
|---|---|---|---|---|---|
| pI 10.32 to 10.15 | 34.1 | 35.5 | 2.1 | 28.3 | 100 |
| pI 10.15 to 9.89 | 15.4 | 38.2 | 5.0 | 41.4 | 100 |

Table 2

|  | N | A-1 | A-2 | A-3 | Total |
|---|---|---|---|---|---|
| pI 10.32 to 10.15 | 19.5 | 32.1 | 1.7 | 46.7 | 100 |
| pI 10.15 to 9.89 | 10.0 | 31.9 | 4.9 | 53.2 | 100 |

Now Examples of the present invention will be given.

Example 1

10 mM of phenylmethylsulfonyl fluoride was added to approximately 350 g of porcine pituitary and 1 liter of a 0.15 M aqueous solution of ammonium sulfate. The resulting mixture was homogenized in a Waring blender for two minutes while cooling. After adjusting the pH value to 4.0, the homogenized mixture was allowed to stand for a sufficient period of time under cooling. Then it was centrifuged and the supernatant was collected. After further adjusting the pH value to 3.0 with a 0.5 M aqueous solution of metaphosphoric acid, the resulting solution was centrifuged and the supernatant was collected. After adjusting the pH value of the supernatant to 6.5 to 7.0, ammonium sulfate was added thereto until 50 % saturation was achieved. Then the mixture was allowed to stand for a sufficient period of time again under cooling. Subsequently it was centrifuged. The precipitate was collected and suspended in 50 to 100 ml of a 0.2 M solution of dibasic potassium phosphate. The suspension was transferred into a dialysis tube and sufficiently dialyzed under cooling. Then the content was taken out and the pH value thereof was again adjusted to 8.5. The obtained material was lyophilized.

The liophilized powder thus obtained was dissolved in a 0.01 M solution of dibasic sodium phosphate and applied to an SP-Sephadex (mfd. by Pharmacia) column equilibrated with the above-mentioned buffer. Fractions eluted with 0.1 M dibasic sodium phosphate were collected and lyophilized.

The lyophilized powder was dissolved in a small amount of a 0.05 M solution of ammonium hydrogencarbonate and fractionated through a molecular sieve by using a Sephadex G-100 (mfd. by Pharmacia) column wherein the same buffer system was employed. Fractions of molecular weights of approximately 40,000 were collected and lyophilized.

The dry powder thus obtained was dissolved in a small amount of a solution of 10 mM Tris-HCl (pH 7.5) and 0.3 M sodium chloride and then fractionated by using a Concanavalin A-Sepharose (mfd. by Pharmacia) column wherein the same buffer system was employed. Fractions eluted with 0.2 M methyl mannoside were collected, sufficiently dialyzed under cooling and then lyophilized. Thus approximately 25 mg of a crude renal growth factor (RGF) was obtained.

Subsequently this crude product was fractionated and purified according to the difference in isoelectric point by using an LKB isoelectric focusing column (mfd. by LKB Produckter AB, Bromma, Sweden; capacity: 110 ml). As carrier ampholytes, Ampholine pH 3.5 - 10 (mfd. by LKB), Ampholine pH 9 - 11 (mfd. by LKB) and Ampholine pH 7 - 9 (mfd. by LKB) were employed. A sorbitol density gradient (5 to 50 %) was prepared and the above-mentioned crude product dissolved in 2 ml of a dense solution was placed onto the center of the column (dense gradient solution: sorbitol 27 g, distilled water 34.9 ml, Ampholine (pH 9 - 11) 2.1 ml, and light gradient solution: sorbitol 2.7 g, distilled water 52.3 ml, Ampholine (pH 9 - 11) 0.3 ml, Ampholine (pH 3.5 - 10) 0.2 ml, Ampholine (pH 7 - 9) 0.2 ml). A 1 M sodium hydroxide solution and a 0.01 M acetic acid solution were used as electrode solutions. Electricity at 800 to 1500 V. was turned on at 4°C for 24 hours. After the completion of the turning of electricity, 1.5 ml portions of the material were collected with a fraction collector. Then the pH value of each fraction at 4°C was immediately determined (COM-11: mfd. by Denki Kagaku Kogyo K.K., electrode type CE 105 C, standard buffer solution: mfd. by Wako Pure Chemicals Co., Inc.). Simultaneously the protein content of each fraction was determined. Fig. 1 shows the chromatographed pattern.

As a result, 104 µg, 281 µg, 415 µg and 103 µg of fractions of a pI higher than 10.32, that of 10.32 to 10.15, that of 10.15 to 9.89 and that lower than 9.89 were obtained respectively from approximately 1.5 mg of the crude renal growth factor.

Each fraction was sufficiently dialyzed under cooling to thereby remove the Ampholines and sorbitol therefrom and then lyophilized to give a powder.

The crude renal growth factor was purified several times by repeating the isoelectric focusing under the same conditions as those employed above. Then fractions of the same pI range were combined together. The mean pI of equine cardiac myoglobin (Type III: mfd. by Sigma Chemical Co.,) used as a standard protein was 7.97.

Evaluation of Renal Growth Promoting Activity:

It was determined which fraction among those obtained above would promote renal growth.

The renal growth promoting effect of renotropin was determined with the guidance of the promotion of the DNA-synthesizing effect of renal cells.

Male CD rats (available from Nippon Charles River Co. and born on the same day (average body weight: 100 g)) were subjected to hypophysectomy and castration and then fed for nine days. After confirming whether the operation had been appropriately carried out or not, the rats were divided into test lots and a control lot each consisting of at least five animals.

50 μg of each fraction fractionated by the above isoelectric focusing was dissolved in 150 μl of a 50 mM borate buffer (pH 7.5) containing 0.1 % of BSA and subcutaneously injected into each rat. After eight hours, the animal was sacrificed by cutting off the head and blood-drawn, and then the right and left kidneys were taken out. After peeling off the capsule, two slices of each kidney were prepared along the corticomedullary axis. These slices were incubated in 2 ml of Krebs-Ringer bicarbonate buffer (pH 7.5) at 37°C for two hours. This buffer had been preliminarily aerated with a gas mixture comprising 95 % of oxygen and 5 % of carbon dioxide and then 2 µ Ci/ml of 1,2-methyl$^3$H-thymidine (mfd. by New England Nuclear Corp.) had been added thereto. After the completion of the incubation, the slices were rinsed with a 4 mM thymidine solution and stored at -20°C.

4 ml of distilled water was added to the stored slices and the resulting mixture was homogenized with the use of an ultradisperser (mfd. by Yamato Kagaku K.K.). Then DNA contained therein was extracted according to a process proposed by A. Fleck and H.M. Munro (cf. Biochem. Biophys. Acta, 55, 571 (1962)). The extracted DNA was hydrolyzed according to a process reported by J.R.W. Wannemacher, J.W.L. Banks and W.H. Wunner (Anal. Biochem., 11, 320 (1965)) and then determined by using bovine thymus DNA (type I, mfd. by Sigma Chemical Co.) as a standard DNA according to a process proposed by K. Burton(Biochem. J. 62,315 (1956)). Simultaneously the radioactivity of 0.5 ml of the extracted DNA was determined in a conventional manner.

The activity of promoting DNA synthesis of the renal cells was determined by radioactivity/total DNA content and expressed in the percentage of each lot by defining that of the control lot as 100 %. As a result, the fraction of a pI higher than 10.32 showed a DNA synthesis promoting activity of 109 %, the fraction of a pI of 10.32 to 10.15 showed that of 130 % (the thick arrow in Fig. 1), the fraction of a pI of 10.15 to 9.89, which showed the largest protein content, showed no DNA synthesis promoting effect (98 %) and the fraction of a pI lower than 9.89 showed that of 115 % (the thin arrow in Fig. 1). Namely, the fraction of a pI of 10.32 to 10.15 and that of a pI lower than 9.89 (9.89 to 9.32) contained a renal growth promoting component. In addition, the fraction of a pI higher than 10.32 also contained the renal growth promoting component, though the activity thereof was slight.

The DNA synthesis promoting effect of the crude renal growth factor of the same batch was determined at the same time. Thus 112 % of the activity was observed.

Effect of Renal Growth Promoting Component on Renal Growth in Vivo:

Male CD-1 mice (available from Nippon Charles River Co. and born on the same day (average body weight: 17 g)) were subjected to hypophysectomy and castration and then fed for nine days. After confirming whether the operation had been appropriately carried out or not, the mice were divided into a test lot and a control lot each consisting of ten animals. 40 μg of the fraction of a pI of 10.32 to 10.15 as obtained above by isoelectric focusing was dissolved in 150 μl of 50 mM borate buffer (pH 7.5) containing a 0.1 % BSA solution. The other 40 μg portions of the fraction of a pI of 10.32 to 10.15 were treated in the same manner. The resulting solution was subcutaneously injected into each animal at a dose of 150 μl/day continuously for five days. On the sixth day, the animal was sacrificed by cutting off the head and blood-drawn, and then the right and left kidneys were taken out. After peeling off the capsule, these kidneys were lyophilized. The ratio of the weight of the dry kidneys to the final body weight of the mouse was calculated and the difference in the kidney weight between the test and control lots were examined. As a result, the test lot showed an increase in the kidney weight of 110 %, which suggests that the employed fraction had a renal growth promoting effect.

Separation and Rebuilding of Subunits $\alpha$ and $\beta$ in Renal Growth Promoting Component:

The fraction of a pI of 10.32 to 10.15 fractionated by isoelectric focusing was subjected to high performance liquid chromatography and subunits thus separated were collected. The fraction was dissolved in a small amount of a 0.1 % aqueous solution of trifluoroacetic acid (TFA) and then separated with a reversed phase column (Baker Bond wide pore butyl $C_4$: mfd. by Baker Research Products) at a linear gradient (10 % - 50 %) with the use of 2-propanol/acetonitrile (7/3) containing 0.1 % of TFA as a solvent.

Thus the subunits α and β were obtained each as three main peaks named $\alpha_1$, $\alpha_2$ and $\alpha_3$; and $\beta_1$, $\beta_2$ and $\beta_3$ in the order of elution. When 3 mg of the sample was separated, 230 µg of $\alpha_1$, 450 µg of $\alpha_2$, 350 µg of $\alpha_3$, 410 µg of $\beta_1$, 570 µg of $\beta_2$ and 60 µg of $\beta_3$ were obtained. As a result of the analysis of amino acid composition, etc., it was found that $\alpha_3$ and $\beta_3$ were luteinizing hormone subunits decomposed to the lowest extent.

Equimolar amounts of $\alpha_1$ and $\beta_1$; $\alpha_2$ and $\beta_2$; and $\alpha_3$ and $\beta_3$ were mixed together. Each mixture thus obtained was maintained at 37°C for 48 hours in a small amount of a 1 % aqueous solution of ammonium carbonate to thereby attempt to associate the subunit α with the subunit β. Subsequently, the mixture was desalted with a TSK gel G 3000 SW column (mfd. by Toyo Soda Mfg. Co., Ltd.) and then lyophilized. The activities of these samples were compared with one another on the basis of the DNA synthesis promoting effects as described above. As a result, the combination of $\alpha_3/\beta_3$

showed the highest activity, i.e., approximately 2.5 times as high as that of the combination of $\alpha_1/\beta_1$ in the difference from the control lot.

Example 2

100 g of an ovine pituitary powder (mfd. by Waitaki NZ Refrigerating Ltd.) was suspended in 400 ml of cold water and then 1.5 liters of a 0.15 M ammonium sulfate solution was added thereto. 10 mM of phenylmethylsulfonyl fluoride was added thereto and the resulting mixture was homogenized with Polytron under cooling. After adjusting the pH value to 4.0, the homogenized mixture was allowed to stand for a sufficient period of time under cooling and then centrifuged. The supernatant was collected and treated in the same manner as described in Example 1. Then it was treated with an SP-Sephadex column, a Sephadex G-100 column and a Concanavalin A-Sepharose column to thereby give a crude renal growth factor.

Further 5 mg of the crude renal growth factor was fractionated with isoelectric focusing, as in Example 1. As a result, a fraction of a pI higher than 9.85, that of a pI of 9.85 to 9.60, that of a pI of 9.60 to 9.10, that of a pI of 9.10 to 8.60, that of a pI of 8.60 to 7.30 and that of a pI lower than 7.30 were obtained. As the result of the same bioassay as described in Example 1, it was confirmed that the fraction of a pI higher than 9.85 contained a main renal growth promoting component.

Example 3

750 ml of an extract of human pituitary (mfd. by KabiVitrun AB) was centrifuged. The resulting precipitate was collected and suspended in 70 ml of a 0.2 M aqueous solution of dibasic potassium phosphate. The resulting suspension was heated to 60°C for three minutes and then dialyzed for two days under cooling. The content was taken out and 0.13 M ammonium sulfate was added thereto. After adjusting the pH value to 4.0, the pH value of the mixture was adjusted again to 3.0 with a 0.5 M metaphosphoric acid solution. Then the mixture was allowed to stand under cooling and centrifuged. The supernatant was collected, sufficiently dialyzed under cooling and then lyophilized.

Then it was treated in the same manner as described in Example 1 with the use of an SP-Sephadex column and a Sephadex G-100 column to thereby give an extract of crude luteinizing hormone.

This crude luteinizing hormone extract was treated with a Concanavalin A-Sephadex column to thereby give a crude renal growth factor.

The obtained crude renal growth factor was fractionated by isoelectric focusing. Each fraction thus obtained was subjected to the same bioassay as described in Example 1. As a result, it was confirmed that a fraction of a pI of 9.05 to 9.90 contained a main renal growth promoting

component.

WHAT IS CLAIMED IS

1. A renal growth promoter comprising luteinizing hormone as an active ingredient.

2. A process for the production of an isomer of luteinizing hormone having a renal growth promoting activity, which comprises fractionating and purifying a crude renal growth factor according to the difference in isoelectric point.

3. A luteinizing hormone which contains 30 % or more neutral glyco-chain component in the glyco-chain component composition of $\beta$ subunit of luteinizing hormone.

4. A renal growth promoter comprising, as an active ingredient, a luteinizing hormone which contains 30 % or more neutral glyco-chain component in the glyco-chain component composition of $\beta$ subunit of luteinizing hormone.

5. A luteinizing hormone which contains 18 % or more neutral glyco-chain component in the glyco-chain component composition of $\beta$ subunit of luteinizing hormone.

6. A renal growth promoter comprising, as an active ingredient, a luteinizing hormone which contains 18 % or more neutral glyco-chain component in the glyco-chain component composition of $\beta$ subunit of luteinizing hormone.

1/1

# FIG. 1

# INTERNATIONAL SEARCH REPORT

International Application No  PCT/JP88/01205

**I. CLASSIFICATION OF SUBJECT MATTER** (if several classification symbols apply, indicate all) [6]

According to International Patent Classification (IPC) or to both National Classification and IPC

Int.Cl$^4$  A61K37/32

**II. FIELDS SEARCHED**

| Minimum Documentation Searched [7] | |
|---|---|
| Classification System | Classification Symbols |
| IPC | A61K37/32 |

| Documentation Searched other than Minimum Documentation to the Extent that such Documents are Included in the Fields Searched [8] |
|---|
| |

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| P | JP, A, 63-264530 (The Calpis Food Industry Co., Ltd.) 1 November 1988 (01. 11. 88) Claim (Family: none) | 1-6 |
| A | Chemical Abstracts Vol. 102, No. 11 Abstract No. 90348W (Chem. Abstr. 102:90348W) 18 March 1985 (18. 03. 85) (Columbus, Ohio, U.S.A.) Nomura K., et. al., Endocrinology, 116(2), p616-21 | 1-6 |
| X | Imahori Kazutomo, Yamakawa Tamio [Sei Kagaku Jiten] 10 April 1984 (10. 04. 84) Tokyo Kagaku Dojin (Tokyo) p209 | 1-6 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 25, 1989 (25. 01. 89) | February 20, 1989 (20. 02. 89) |
| International Searching Authority | Signature of Authorized Officer |
| Japanese Patent Office | |